Europäisches Patentamt

European Patent Office

Office européen des brevets

(11) Publication number: **0 544 372 A1**

## EUROPEAN PATENT APPLICATION

(21) Application number: 92203596.9

(51) Int. Cl.⁵: **B01J 27/19**, C07C 5/48

(22) Date of filing: 21.11.92

(30) Priority: **26.11.91 IT MI913150**

(43) Date of publication of application:
**02.06.93 Bulletin 93/22**

(84) Designated Contracting States:
**AT BE CH DE DK ES FR GB GR IE LI LU MC NL PT SE**

(71) Applicant: **ENIRICERCHE S.p.A.**
**Corso Venezia 16**
**I-20121 Milan(IT)**

(72) Inventor: **Kutyrev, Michail**
**Via Morandi 2/c**
**I-20097 San Donato Milanese Milan(IT)**
Inventor: **Cavani, Fabrizio**
**Viale Risorgimento 4**
**I-40136 Bologna(IT)**
Inventor: **Trifiro', Ferruccio**
**Via Longhena, 6**
**I-40139 Bologna(IT)**

(74) Representative: **Roggero, Sergio et al**
**Ing. Barzanò & Zanardo Milano S.p.A. Via Borgonuovo 10**
**I-20121 Milano (IT)**

(54) **Catalyst and process for oxidative dehydrogenation of ethane into ethylene.**

(57) A catalyst for oxidative dehydrogenation can be defined by means of the formula:

$$Mo_aP_1X_bY_cZ_dQ_eO_f \quad (I)$$

wherein:

X  represents an element selected from sodium, potassium rubidium, cesium, calcium and barium, or is ammonium;

Y  represents antimony, or a combination of antimony and tungsten;

Z  represents an element selected from iron, cobalt, nickel, vanadium, niobium, tin, zirconium or zinc, or one of these elements in combination with chromium and/or copper;

Q  represents a rare-earth element;

a  is a numeral comprised within the range of from 6 to 12;

b  is a numeral comprised within the range of from 0.1 to 6;

c  is a numeral comprised within the range of from 0.1 to 6;

d  is a numeral comprised within the range of from 0.01 to 3;

e  is a numeral comprised within the range of from 0.01 to 5;

f  is a suitable numeral for satisfying the valency requirements of the other elements.

A process is disclosed for producing ethylene by means of ethane oxidative dehydrogenation, using catalyst (I).

Rank Xerox (UK) Business Services
(3.10/3.6/3.3.1)

The present invention relates to an oxidative dehydrogenation catalyst, to the process for preparing it and to the use thereof in the processes for ethylene production by means of oxidative ethane dehydrogenation.

Crystalline catalyst which are heteropolyoxymetallated or heteropolycompounds ("HPC" in the following) are known, which have a complex structure and generally are constituted by a primary structural unit, said "Keggin unit", constituted by a cage anion containing a tetracoordinated central atom (usually phosphorus, but it may also be silicon or aluminum) and twelve atoms of transition metals with octahedral coordination (usually molybdenum, but also tungsten and, partly, vanadium can be present). The counterion usually is constituted by protons or alkali metals, but introducing different metal types is also possible.

The particular structure of HPC makes them active for reactions of acidic catalysed type (thanks to the protonic function), as well as for reactions of redox type (thanks to the presence of metals with variable valency state). Therefore, HPC compounds find application at industrial level in the synthesis of methacrylic acid from isobutene, but they are reported to be also active in the reactions of alkylation of aromatic systems, as well as reactions of selective oxidation of unsaturated systems, such as the conversion of butene into maleic anhydride and crotonic aldehyde into furan. For a review of the catalytic properties of HPC reference is made to Misono, Catal. Rev. Sci. Eng., (1987) 269.

The application of HPC as heterogeneous catalysts for vapour phase reactions is generally limited by the inherent stability of HPC. In fact, such systems undergo decomposition at temperatures higher than 350-400°C. The decomposition of the structure can even start at lower temperatures than 300°C (G.A. Tsigdinos, Topics Curr. Chem., V 76, pages 1-66, Springer Verlag 1978).

The present Applicant has found now, according to the present invention, that the addition of particular metals to HPC, in particular to HPC stabilized with antimony-(V), makes it possible catalysts to be obtained which are endowed with an exceptionally good thermal stability, besides high activity and selectivity values in the reaction of oxidative dehydrogenation of ethane into ethylene.

In accordance therewith, in a first aspect thereof, the present invention relates to a heat-stable, active and selective catalyst for oxidative ethane dehydrogenation, which can be defined by means of the formula:

$$Mo_a P_1 X_b Y_c Z_d Q_e O_f \qquad (I)$$

wherein:

X    represents an element selected from sodium, potassium rubidium, cesium, calcium and barium, or is ammonium;

Y    represents antimony, or a combination of antimony and tungsten;

Z    represents an element selected from iron, cobalt, nickel, vanadium, niobium, tin, zirconium or zinc, or one of these elements in combination with chromium and/or copper;

Q    represents a rare-earth element;

a    is a numeral comprised within the range of from 6 to 12;

b    is a numeral comprised within the range of from 0.1 to 6;

c    is a numeral comprised within the range of from 0.1 to 6;

d    is a numeral comprised within the range of from 0.01 to 3;

e    is a numeral comprised within the range of from 0.01 to 5;

f    is a suitable numeral for satisfying the valency requirements of the other elements.

In the preferred form of practical embodiment, in the above formula (I):

X    represents potassium, cesium, barium or ammonium;

Y    represents antimony;

Z    represents iron, cobalt or nickel, or one of these elements in combination with chromium;

Q    represents cerium, lanthanum or praseodymium;

a    is a numeral comprised within the range of from 10 to 12;

b    is a numeral comprised within the range of from 1.5 to 4;

c    is a numeral comprised within the range of from 0.5 to 3.5;

d    is a numeral comprised within the range of from 0.5 to 2;

e    is a numeral comprised within the range of from 0.5 to 3.5.

In the most preferred form of practical embodiment:

X    represents potassium;

Y    represents antimony;

Z    represents iron, or a combination of iron and chromium;

Q    represents cerium.

The catalyst according to the present invention is hence a catalyst of HPC type, as defined hereinabove, stabilized by the presence of antimony with oxidation level (V), to which the other catalytic metals have been added, so as to couple, With the exceptionally high heat stability, also good characteristics of activity and selectivity in the reaction of oxidative dehydrogenation of ethane into ethylene.

According to another aspect thereof, the present invention relates to a process for preparing catalyst (I), which generally comprises the following steps:

- preparation of an aqueous solution of molybdenum, phosphorus compounds, and of X and Y elements;
- preparation of an aqueous solution of the compounds of elements Z and Q;
- mixing the resulting solutions With each other, until a homogeneous mixture is obtained, with the addition of an oxidizer agent which makes it possible antimony to be present in the oxidation state (V);
- evaporation of water from the homogeneous mixture in order to obtain a solid; and
- calcination of the resulting solid at temperatures comprised within the range of from 180°C to 800°C, for a time of from 0.5 to 48 hours.

Examples of compounds of compounds of molybdenum, phosphorus, and elements X and Y are used: ammonium paramolybdate $(NH_4)_6Mo_7O_{24}.4H_2O$; dipotassium monohydrogen phosphate $K_2HPO_4$; potassium nitrate $KNO_3$; $KSbOC_2H_4O_6.5H_2O$; $5(NH_4)_2O\ 12WO_3.5H_2O$ and $(NH_4)H_2PO_4$.

Examples of compounds of elements Z and Q are: iron nitrate $Fe(NO_3)_3.9H_2O$, ammonium vanadate $NH_4VO_3$, chromium nitrate and cerium nitrate.

Advantageously, an aqueous solution of the compounds of molybdenum, phosphorus and elements X and Y, is prepared by operating with stirring at a temperature of the order of 80-90°C, and an aqueous solution of the compounds, preferably nitrates, of elements Z and Q, are prepared by operating at 30-40°C. The resulting solutions are mixed with each other, and an oxidizer agent is added which makes it possible antimony, or at least most antimony, to be brought, in the end catalyst obtained, to oxidation state (V). The preferred oxidizer agent is nitric acid. Then, water is evaporated off by operating up to a temperature of the order of 140°C, in order to obtain a solid material which is suitably pelletized. The calcination step is preferably carried out by gradually heating the desiccated and pelletized solid material from the drying temperature up to the calcination temperature. A typical temperature profile is as follows: 180°C for 10 hours, 200°C for 6 hours, 220°C for 6 hours, 250°C for 2 hours, 300°C for 2 hours and 350°C for 6 hours.

The present invention also relates to a process for preparing ethylene, by means of oxidative ethane dehydrogenation, carried out in the presence of catalyst (I). The process can be carried out in a reaction vessel for oxidative dehydrogenation, with the catalyst being in the form of a fixed bed, by feeding a mixture, in the gas phase, containing ethane and oxygen, with an ethane content of from 1 to 90% by volume in the same mixture. An inert diluent can be present, such as, e.g., nitrogen. In the reactor, the process is advantageously carried out in the gas phase, at a temperature comprised within the range of from 400 to 600°C, under the atmospheric pressure, or under a pressure higher than atmospheric pressure, of, e.g., up to 5 abs. atm., and with a contact time of from 0.1 to 20 seconds. Under these conditions, a good ethane conversion per passage, with a high selectivity, to ethylene, is obtained. Ethylene can be recovered from the products from the gas stream leaving the reactor, and unconverted ethane can be recycled.

The following experimental examples are reported in order to better illustrate the invention.

Example 1 (Comparison Example)
‒ ‒ ‒ ‒ ‒ ‒

200 ml of water is prepared in a glass flask equipped with stirrer and heating plate and then 13.9 g of $K_2HPO_4$, 8.1 g of $KNO_3$ and 169.5 g of ammonium paramolybdate $(NH_4)_6Mo_7O_{24}.4H_2O$ are dissolved after each other in the sequence stated, at a temperature of 60-80°C. The solvent is evaporated, and the residue is dried at the temperature of 140°C for 16 hours. The resulting solid material is pelletized, in order to convert it into particles of 0.5-1 mm of diameter and is subsequently calcined at the following temperatures: 180°C for 10 hours, 200°C for 6 hours, 220°C for 6 hours, 250°C for 2 hours, 300°C for 2 hours and 350°C for 6 hours.

The resulting catalyst, having the composition $K_3P_1Mo_{12}$, as referred to metals, results to be stable up to 400°C; at higher temperatures, it undergoes decomposition; the decomposition can be monitored by means of such techniques as differential calorimetry, thermogravimetry, and verified by X-ray diffractometry and IR spectroscopy; the decomposition of the structure is accompanied by the decay of catalytic activity.

Example 2 (Comparison Example)

200 ml of water is prepared in a glass flask and then 13.9 g of $K_2HPO_4$, 8.1 g of $KNO_3$ and 155.5 g of ammonium paramolybdate $(NH_4)_6Mo_7O_{24}.4H_2O$ are dissolved after each other in the sequence stated, at a temperature of 60-80°C. A second solution containing 32.3 g of $Fe(NO_3)_3.9H_2O$ is prepared, and is added dropwise to the first solution. The resulting mixture is evaporated, is dried and calcined according to the same modalities as reported in Example 1.

The resulting catalyst, having the composition $K_3P_1Mo_{11}Fe_1$, as referred to metals, undergoes decomposition at higher temperatures than 450°C, with decay of catalytic activity.

Example 3 (Comparison Example)

The process is carried out as in Example 2, with the difference that the second solution is constituted by 9.4 g of $NH_4VO_3$ in 200 ml of water.

The resulting catalyst, having the composition $K_3P_1Mo_{11},V_1$, as referred to metals, is stable up to 400°C; at higher temperatures, the catalytic activity decreases.

Example 4 (Comparison Example)

200 ml of water is prepared in a glass flask and then 13.9 g of $K_2HPO_4$, 155.4 g of ammonium paramolybdate $(NH_4)_6Mo_7O_{24}.4H_2O$ and 26.7 g of $KSbOC_2H_4O_6.5H_2O$ are dissolved after each other in the sequence stated, at a temperature of 60-80°C. The resulting mixture is treated as in Example 1. In this preparation, the end oxidation state of antimony mainly is trivalent. A catalyst having the composition $K_3P_1Mo_{11}Sb_1$, as referred to metals, is obtained, which undergoes decomposition at higher temperatures than 400°C.

Example 5 (Comparison Example)

200 ml of water is prepared in a glass flask and then 13.9 g of $K_2HPO_4$, 155.4 g of ammonium paramolybdate $(NH_4)_6Mo_7O_{24}.4H_2O$ and 26.7 g of $KSbOC_2H_4O_6.5H_2O$ are dissolved after each other in the sequence stated, at a temperature of 60-80°C.

To the resulting solution, 25 ml of conc. $HNO_3$ (65%) is then added dropwise. The resulting mixture is treated as in Example 1. Owing to the presence of an oxidizer agent during the preparation, the end oxidation state of antimony is mainly (V). A catalyst having the composition $K_3P_1Mo_{11}Sb_1$, as referred to metals, is obtained. The tests performed in thermobalance and by differential calorimetry, as well as by IR and X-ray techniques, evidence that the structure is stable up to 670°C.

Example 6 (Comparison Example)

200 ml of water is prepared in a glass flask and then 13.9 g of $K_2HPO_4$, 155.4 g of ammonium paramolybdate $(NH_4)_6Mo_7O_{24}.4H_2O$ and 25.3 g of $5(NH_4)_2O\ 12WO_3.5H_2O$ are dissolved after each other in the sequence stated, at a temperature of 60-80°C. The resulting mixture is treated as in Example 1. A catalyst having the composition $K_3P_1Mo_{11}W_1$ is obtained. The catalyst is stable up to 450°C; higher temperatures cause the decomposition thereof, and the complete decay of activity.

The catalysts of Examples 1 - 6 are submitted to the tests of catalytic activity in oxidative ethane dehydrogenation, by operating With a contact time of 2.7 seconds, and a feed of 3.6% ethane in air.

In these tests, a laboratory equipment is used, which consists of a tubular microreactor (inner diameter 5 mm, height 20 cm), inside a copper tube which in its turn is immersed inside heating resistors. The amount of catalyst charged to the reactor for the tests is of approximately 3 grams. An axial thermocouple immersed inside the catalytic bed makes it possible the reaction temperature to be measured, and the presence to be verified of axial temperature gradients. The catalyst is prepared in the suitable morphology by compression and granulation, with the end size of the granules being comprised within the range of from 0.2 to 0.5 mm. The reactant mixture is preheated and then is fed to the reactor from top downwards (down-flow mode). The mixture of reaction products is sent to a sampling valve, contained inside an oven kept at 200°C, and is injected to a first gaschromatograph equipped with a flame-ionization detector, in order to detect ethane, ethylene end, possibly, other partial oxidation products. The column used to separate the products is a 30 cm long Porapak QS column. The oven of the chromatograph is scheduled from 40°C (10 minutes of initial isotherm) to 150°C, with a gradient of 16°C/minute. After the first sampling valve, the mixture of the products is sent to a second gaschromatograph, equipped with a heat-conductivity detector, in order to detect the presence of oxygen and carbon oxides. The column used for the separation is packed with Carbosieve S and is 2 metres long. The temperature schedule starts from 40°C (5 minutes of isotherm), with a subsequent temperature increase rate of 16°C/minute, up to 240°C.

The results of activity tests are reported in the following Table 1.

Table 1

| Catalyst Example No. | | Temperature (°C) | Ethane conversion (%) | Selectivity to ethylene (%) | Ethylene yield (%) |
|---|---|---|---|---|---|
| 1 | Fresh | 425 | 5.6 | 42.6 | 2.4 |
|   | After 3 hrs | 425 | 3.8 | 40.2 | 1.5 |
| 2 | Fresh | 425 | 5.4 | 59.0 | 3.3 |
|   | Fresh | 480 | 8.5 | 50.2 | 4.3 |
|   | Fresh | 500 | 9.7 | 48.2 | 4.7 |
|   | After 3 hrs | 500 | 7.2 | 51.4 | 3.7 |
| 3 | Fresh | 425 | 24.0 | 21.9 | 5.3 |
|   | After 3 hrs | 425 | 11.2 | 29.2 | 3.3 |
| 4 | Fresh | 425 | 4.8 | 44.2 | 2.1 |
|   | After 3 hrs | 425 | 3.9 | 45.1 | 1.8 |
| 5 | Fresh | 425 | 2.8 | 42.5 | 1.2 |
|   | Fresh | 540 | 6.7 | 33.5 | 2.2 |
|   | After 10 hrs | 540 | 6.1 | 38.2 | 2.3 |
| 6 | Fresh | 425 | 5.3 | 45.1 | 2.4 |
|   | Fresh | 450 | 6.1 | 39.1 | 2.4 |
|   | After 3hrs. | 450 | 3.8 | 44.2 | 1.7 |

The experimental examples reported hereinabove clearly indicate that antimony, added to HPC in its oxidation state (V), supplies stability to said HPC. However, the catalytic activity and selectivity to ethylene result to be still low.

Therefore, by using the catalyst prepared in Example 5 ($K_3P_1Mo_{11}Sb_1$) as the base matrix, other catalysts containing different metal types are prepared by adding to the main solution, a second solution containing the proper amounts of a water-soluble salt of the selected metal.

In that way, the following catalysts are prepared:

Example 7 : $K_3P_1Mo_{11}Sb_1V_1$ (comparison catalyst)

Example 8 : $K_3P_1Mo_{11}Sb_1Fe_1$ (comparison catalyst)

Example 9 : $K_3P_1Mo_{11}Sb_1Fe_1Ce_{0.25}$

Example 10: $K_3P_1Mo_{11}Sb_1Fe_1Ce_{0.25}Cr_{0.5}$

Example 11: $K_3Sb_1Mo_{11}Fe_1Ce_{0.25}Cr_{0.5}$ (comparison catalyst)

Example 12: $K_3P_1Sb_1Mo_{11}Fe_1Ce_{1.5}Cr_{0.5}$

Example 13: $K_1P_1Sb_1Mo_{11}Fe_1Ce_{0.25}Cr_{0.5}$

Example 14: $K_1P_1Sb_1Mo_{11}Fe_1Ce_{0.66}Cr_{0.5}$

The catalysts of Examples 7 - 11 are submitted to the tests of oxidative ethane dehydrogenation and the results are reported in following Table 2.

All the tests are carried out with an ethane concentration in air of 3.6%;

Table 2

| Catalyst Example No. | Time (sec) | Temperature (°C) | Ethane conversion % | Selectivity to ethylene % * | Ethylene yield % | Remarks |
|---|---|---|---|---|---|---|
| 5 | 2.7 | 500 | 5.9 | 37.8 | 2.2 | (a) |
| 7 | 2.7 | 430 | 6.8 | 46 | 3.1 | (b) |
| 7 | 2.7 | 500 | 14.3 | 24 | 3.4 | (b) |
| 8 | 2.7 | 540 | 6.8 | 83 | 5.6 | (c) |
| 9 | 2.7 | 540 | 9.6 | 65 | 6.2 | (c) |
| 10 | 2.7 | 540 | 17.2 | 62 | 10.7 | (c) |
| 10 | 10 | 540 | 36.2 | 54 | 19.6 | (d) |
| 10 | 10 | 540 | 26.1 | 74 | 19.3 | (e) |
| 10 | 10 | 540 | 26.1 | 74 | 19.3 | (f) |
| 11 | 10 | 500 | 9.2 | 22 | 2.0 | (g) |

EP 0 544 372 A1

(*): The balance to 100 of selectivity is only constituted by carbon oxides;

(a): Antimony added as Sb-(V);

(b): $CO/CO_2$ = 3 - 5/1;

(c): $CO/CO_2$ = 1/3 - 6;

(d): Fresh catalyst;

(e): After 10 hours;

(f): After 150 hours;

(g): Fresh catalyst, catalyst prepared without P, therefore not characterized by a structure of HPC.

The catalyst of Example 10 is submitted to some tests of ethane dehydrogenation, by operating at 510°C, with a contact time of 2.7 seconds, a 3.6% concentration of ethane in the feed, and by varying the content of oxygen in the feed stream. The data reported in the following Table 3 are those which were detected after a 20-hour reaction time and indicate that the selectivity to ethylene is strongly conditioned by the oxygen content in the feed stream. In fact, when the concentration of oxygen is decreased from 9% down to 2%, the formation of carbon oxides is strongly reduced, with a consequent increase in selectivity to ethylene; on the contrary, ethane conversion does not result to be changed.

Table 3

| Test No. | $O_2$ (%) | Ethane conversion % | Selectivity to ethylene % | Ethylene yield % |
|---|---|---|---|---|
| 1 | 9 | 10.7 | 76 | 8.1 |
| 2 | 7 | 10.6 | 78 | 8.3 |
| 3 | 4 | 10.8 | 90 | 9.7 |
| 4 | 2 | 10.7 | 98 | 10.5 |

The catalysts of Examples 10 - 14 are submitted to the tests of oxidative ethane hydrogenation by operating with an oxygen concentration of 10%, and under the other conditions as reported in Table 4.

Table 4 also displays the values of % ethane conversion and % selectivity to ethylene, at the reaction times set forth in the same Table.

In any case, the reaction byproducts are essentially constituted by carbon dioxide.

Table 4

| Catalyst Example No. | Reaction conditions | | | Results | |
|---|---|---|---|---|---|
| | Temp. ($\varrho$C) | Time (sec) | Ethane concentration % | Ethane conversion % | Selectivity to ethylene % |
| 10 ( 6 h) | 550 | 14 | 25 | 16 | 68 |
| 10 ( 6 h) | 550 | 25 | 25 | 21 | 65 |
| 10 (25 h) | 550 | 12 | 25 | 11.8 | 64 |
| 12 ( 6 h) | 550 | 4.2 | 25 | 10 | 65 |
| 12 ( 6 h) | 550 | 12 | 25 | 16.9 | 56 |
| 12 (25 h) | 550 | 4.2 | 25 | 9.6 | 69 |
| 12 (25 h) | 550 | 12.4 | 25 | 16.2 | 54 |
| 13 ( 6 h) | 470 | 5.2 | 35 | 9.8 | 94 |
| 13 ( 6 h) | 470 | 13 | 35 | 16.3 | 88 |
| 13 (25 h) | 470 | 13 | 35 | 11.8 | 89 |
| 14 ( 6 h) | 450 | 7.6 | 35 | 10.8 | 89 |
| 14 ( 6 h) | 490 | 12 | 35 | 20.1 | 78 |
| 14 (40 h) | 490 | 12 | 35 | 17.8 | 81 |

**Claims**

1. Catalyst for oxidative dehydrogenation, which can be defined by means of the formula:

$$Mo_aP_1X_bY_cZ_dQ_eO_f \qquad (I)$$

wherein:

X     represents an element selected from sodium, potassium rubidium, cesium, calcium and barium, or is ammonium;

Y     represents antimony, or a combination of antimony and tungsten;

Z     represents an element selected from iron, cobalt, nickel, vanadium, niobium, tin, zirconium or zinc, or one of these elements in combination with chromium and/or copper;

Q     represents a rare-earth element;

a     is a numeral comprised within the range of from 6 to 12;

b     is a numeral comprised within the range of from 0.1 to 6;

c     is a numeral comprised within the range of from 0.1 to 6;

d     is a numeral comprised within the range of from 0.01 to 3;

e     is a numeral comprised within the range of from 0.01 to 5;

f     is a suitable numeral for satisfying the valency requirements of the other elements.

2. Catalyst according to claim 1, characterized in that in Formula (I):

x     represents potassium, cesium, barium or ammonium;

Y     represents antimony;

Z     represents iron, cobalt or nickel, or one of these elements in combination with chromium;

Q     represents cerium, lanthanum or praseodymium;

a     is a numeral comprised within the range of from 10 to 12;

b     is a numeral comprised within the range of from 1.5 to 4;

c     is a numeral comprised within the range of from 0.5 to 3.5;

d     is a numeral comprised within the range of from 0.5 to 2;

e     is a numeral comprised within the range of from 0.5 to 3.5.

3. Catalyst according to claim 2, characterized in that, in said Formula (I):

X     represents potassium;

Y     represents antimony;

Z     represents iron, or a combination of iron and chromium;

Q     represents cerium.

**4.** Process for preparing catalyst (I) according to claims 1 - 3, characterized in that it comprises the following steps:
- preparation of an aqueous solution of molybdenum, phosphorus compounds, and of X and Y elements;
- preparation of an aqueous solution of the compounds of elements Z and Q;
- mixing the resulting solutions with each other, until a homogeneous mixture is obtained, with the addition of an oxidizer agent which makes it possible antimony to be present in the oxidation state (V);
- evaporation of water from the homogeneous mixture in order to obtain a solid; and
- calcination of the resulting solid at temperatures comprised within the range of from 180°C to 800°C, for a time of from 0.5 to 48 hours.

**5.** Process according to claim 4, characterized in that the following compounds of compounds of molybdenum, phosphorus and elements X and Y are used: ammonium paramolybdate $(NH_4)_6Mo_7O_{24}.4H_2O$; dipotassium monohydrogen phosphate $K_2HPO_4$; potassium nitrate $KNO_3$; $KSbOC_2H_4O_6.5H_2O$; $5(NH_4)_2O.12WO_3.5H_2O$ and $(NH_4)H_2PO_4$.

**6.** Process according to claim 4, characterized in that the following compounds of elements Z and Q are used: iron nitrate $Fe(NO_3)_3.9H_2O$, ammonium vanadate $NH_4VO_3$, chromium nitrate and cerium nitrate.

**7.** Process according to claims 4 - 6, characterized in that an aqueous solution of the compounds of molybdenum, phosphorus and of elements X and Y, by operating with stirring at a temperature of the order of 80-90°C, and an aqueous solution of the compounds, preferably nitrates, of elements Z and Q, by operating at 30-40°C, are prepared; the resulting solutions are mixed with each other, an oxidizer agent and preferably nitric acid is added, and water is evaporated off by operating up to a temperature of the order of 140°C, in order to obtain a solid which is pelletized and gradually heated from the drying temperature up to the calcination temperature.

**8.** Process for preparing ethylene, by means of oxidative ethane dehydrogenation, characterized in that it is carried out in the presence of catalyst (I) according to claims 1 - 3.

**9.** Process according to claim 8, characterized in that it is carried out with the catalyst being in the form of a fixed bed, by feeding a mixture, in the gas phase, containing ethane and oxygen, with an ethane content of from 1 to 90% by volume in the same mixture, possibly diluted with an inert gas, by operating in gas phase, at a temperature of from 400 to 600°C, under a pressure of from atmospheric pressure up to 5 abs. atm. and with a contact time of from 0.1 to 20 seconds.

European Patent
Office

**EUROPEAN SEARCH REPORT**

Application Number

EP   92 20 3596

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 253 896 (MITSUBISHI RAYON)<br>* claims 1,3 *<br>--- | 1-7 | B01J27/19<br>C07C5/48 |
| X | EP-A-0 424 900 (MITSUBISHI RAYON)<br>* claims 1,2; example 12 *<br>--- | 1-7 | |
| X | EP-A-0 350 862 (MITSUBISHI RAYON)<br>* claim 1; examples 7,8 *<br>--- | 1-7 | |
| A | FR-A-2 247 438 (STANDARD OIL)<br>* claims 1-9 *<br>--- | 8,9 | |
| A | US-A-4 524 236 (UNION CARBIDE)<br>* the whole document *<br>--- | 8-9 | |
| P,X | EP-A-0 467 144 (BASF)<br>* claims 1,5 *<br>--- | 1-7 | |
| X | EP-A-0 454 376 (MISTUBISHI RAYON)<br>* claims 1-8 *<br>--- | 1-7 | |
| P,X | EP-A-0 486 291 (MITSUI TOATSU CHEMICALS)<br>* the whole document *<br>----- | 1-7 | TECHNICAL FIELDS SEARCHED (Int. Cl.5 )<br><br>B01J<br>C07C |

The present search report has been drawn up for all claims

| Place of search | Date of completion of the search | Examiner |
|---|---|---|
| THE HAGUE | 01 MARCH 1993 | MICHIELS P. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)